Europäisches Patentamt

(19) European Patent Office

Office européen des brevets

(11) Publication number: **0 049 012**
**B1**

(12) **EUROPEAN PATENT SPECIFICATION**

(45) Date of publication of patent specification: **06.11.85**

(21) Application number: **81201026.2**

(22) Date of filing: **14.09.81**

(51) Int. Cl.⁴: **C 12 P 19/04,  C 12 P 19/06,**
**C 09 K 7/00,  C 08 B 37/00,**
**B 01 D 13/00,  E 21 B 43/22**

(54) **Treatment of pseudoplastic polysaccharide solutions.**

(30) Priority: **29.09.80 GB 8031404**

(43) Date of publication of application:
**07.04.82 Bulletin 82/14**

(45) Publication of the grant of the patent:
**06.11.85 Bulletin 85/45**

(84) Designated Contracting States:
**BE CH DE FR GB IT LI NL SE**

(56) References cited:
**DE-A-2 737 989**
**US-A-3 541 006**
**US-A-4 010 071**

**Food Collpids (H.D. GRAHAM) (1977) p. 507**

The file contains technical information
submitted after the application was filed and
not included in this specification

(73) Proprietor: **SHELL INTERNATIONALE**
**RESEARCH MAATSCHAPPIJ B.V.**
**Carel van Bylandtlaan 30**
**NL-2596 HR Den Haag (NL)**

(72) Inventor: **Van Lookeren Campagne, Constant**
**Johan**
**Carel van Bylandtlaan 30**
**NL-2596 HR The Hague (NL)**
Inventor: **Roest, Jacob Bernard**
**Badhuisweg 3**
**NL-1031 CM Amsterdam (NL)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## 0 049 012

**Description**

The present invention relates to a process for the concentration of polysaccharide-containing solutions which can be used in supplementary oil recovery. Polysaccharides produced by fermentation of carbohydrates using microorganisms, such as Xanthan gum produced by *Xanthomonas*-type bacteria, are well-known but either available in the form of powders or as diluted aqueous solutions. Although a powder is much preferred from a logistic point of view over shipping a diluted solution, powders have some inherent handling disadvantages such as high hygroscopicity and especially the difficulty to (re)dissolve them in water. Also the quality of the product may deteriorate during storage due to damaged structure of the product caused by oxidation during drying. These disadvantages, and the logistic disadvantages of transporting a dilute solution, can be minimised by the use of a concentrated aqueous solution. From US 3,541,006 it is known to concentrate aqueous solutions of polysaccharides having a molecular weight of about 39500 by ultrafiltration. However in order to achieve a satisfactory ultrafiltration turbulence promoters like elastomeric particles are required to negate serious quantitive limitations on the mass-transport through the membranes.

The viscosity of *Xanthomonas*-produced polysaccharides is very high (e.g. in the order of 7000 mPa.s or even more for a 2% wt/wt aqueous solution which makes them very difficult substances to be subjected to ultrafiltration-techniques. Moreover, products produced by microorganisms usually contain materials such as cellular debris which one would expect to clog filtration membranes and lead to a high consumption of energy, with early replacement of pumping equipment, and also to very low fluxes. Surprisingly, it has been found that highly viscous, diluted water-soluble pseudoplastic polysaccharide-containing solutions, even when containing cellular debris, can be concentrated by ultrafiltration without any substantial fouling of the membranes and with acceptable fluxes. Useful concentration can be obtained by ultrafiltration of polysaccharide containing aqueous solutions like fermentation broths having a viscosity of at least 1900 mPa.s, and fermentation broths having an initial viscosity of even 4000 mPa.s can still be handled satisfactorily.

The ultrafiltration concentration process of the present invention can be carried out using ultrafiltration techniques and equipment known to those skilled in the art, subject, of course, to the use of a porous membrane whose pores are sufficiently small to prevent passage of the polysaccharide through the membrane. Suitable membrane materials, including the commercially available polysulphone and polyvinylidene fluoride membranes, have a mol. wt. cut off (i.e. the maximum molecular weight of product which can pass through the membrane) of at most 65,000. The membrane is, of course, supported on a backing plate which protects it from any physical strain imposed by the ultrafiltration procedure. Normally, the ultrafiltration process will be carried out under a positive pressure in the range of from 2 to 20 bar, preferably in the range of from 4 to 10 bar. To achieve a satisfactory result, the pumping rate with which the polysaccharide solution is passed over the mebrane should be at least 0.28 m/s. Without being bound to any particular theory, it is believed that the possession of pseudoplastic, i.e. shear-thinning, viscosity properties is at least in part responsible for the surprising degree of concentration/viscosity that can be attained by ultrafiltration, and hence the present process is applicable to polysaccharides having such properties.

Thus, the present invention relates to a process for the concentration of an aqueous solution of a water-soluble pseudoplastic polysaccharide, having a viscosity of at least 1900 mPa.s (determined at 25°C), which comprises ultrafiltration of such a solution by contacting the solution with a porous membrane, having a molecular weight cut-off of at most 65,000, at a pumping rate of at least 0.28 m/s and at a pressure differential over said membrane of 2—20 bar (g), and withdrawing liquid which has permeated through the membrane.

Preferably, in order to achieve a reasonable biopolymer concentration in the retentate, the pumping rate is not more than 0.60 m/s. The membrane has preferably a molecular weight cut-off of not more than 20,000. The present process is particularly useful to concentrate polysaccharide aqueous solutions having a viscosity of at least 7000 mPa.s and is therefore preferably used to concentrate polysaccharide solutions of that viscosity level.

The highly viscous polysaccharide solutions can be subjected to ultrafiltration suffering only low pressure drops which means that high fluxes can be obtained and maintained. Suitable ultrafiltration equipment includes tubular membranes, spiral wound membranes, and plate and frame membranes; the last has generally given the best overall results, but the preferred system in any specific instance will be dictated to a large extent by the mechanical constraints and space limitations in relation to the volume of material to be treated and the area of membrane required. Preferably the polysaccharide solution is recirculated over the porous membrane in a direction parallel to the plane of the membrane.

The aqueous polysaccharide solutions which can be concentrated by the process of the present invention can be obtained by dissolving suitable water-soluble polysaccharides in water; alternatively, and often more conveniently, they can be the actual broth resulting from the fermentation of a polysaccharide-producing microorganism such as *Xanthomonas* sp. The immediate product of the fermentation of a suitable nutrient medium (containing assimilable carbon sources, such as starch, glucose or wheat bran, and assimilable nitrogen sources such as ammonium salts, yeast extract or defatted soybean meal) with microorganisms (e.g. conventional *Corynebacterium* or *Xanthomonas* bacteria such as

2

# 0 049 012

*Xanthomonas compestris* ATCC 13951 or B 1459, or *Pseudomonas* sp. such as NCIB 11264 or 11592) is a fermentation broth containing the polysaccharide, the microbe which produced it, and other materials including various inorganic ions such as phosphate, nitrate, potassium and magnesium.

The fermentation broth will normally contain cellular debris, as well as nonviable bacteria, which may present difficulty in field application of the polymer flood in that plugging of the sediment and injection water filters can occur. Methods have already been suggested in the literature to clarify aqueous solutions of polysaccharides containing cellular debris; e.g. by enzymatic treatment (US 3,966,610 and US 4,010,071) which may be combined with absorbing the material on solid siliceous material (United States Patent 4,119,491), or by caustic or flocculation techniques. Any method which is intended to clarify a fermentation broth can advantageously be used in conjunction with the ultrafiltration concentration of a polysaccharide-containing fermentation broth. Such a combination of techniques will lead to a concentrated and clarified polysaccharide solution.

It has been found that the combination of ultrafiltration and enzymatic treatment gives a valuable combination of good clarification allied with concentration. Accordingly, a preferred embodiment of the present invention is a process for concentrating and clarifying a viscous, aqueous solution of a water-soluble polysaccharide solution by subjecting the solution to ultrafiltration and also to the action of a cell-lytic enzyme. The enzyme may be added either before or after the ultrafiltration treatment. In one convenient embodiment, described in more detail hereinafter, the enzyme is present in the ultrafiltration retentate which is recirculated through the UF system, thereby enabling those enzymatic breakdown products of sufficiently low molecular weight to pass through the membrane and be removed in the permeate.

The enzyme to be used in the enzymatic treatment should be capable of degrading microbial cells or cellular debris to such an extent that the polysaccharide solution is clarified without substantial loss of viscosity. Enzymes which can be suitably used in this treatment comprise amongst others "Alcalase" and "Esperase" *(ex NOVO) "Maxazyme" and "Maxatase" (ex Gist-Brocades) and "Kitalase" (ex Kumiai). Especially good results can be obtained using "Esperase" as the enzyme having cell-lytic activity.

The enzymatic treatment can be carried out by methods known the art. For instance, the enzyme or an enzyme-containing solution can be added in a minor amount to an aqueous fermentation broth containing polysaccharide and cellular debris and the mixture allowed to stand, with or without agitation, for a sufficient time to allow the enzymatic action to achieve a sufficient degradation and dissolution of the cellular material. The concentration of enzyme employed will vary with the concentration of the polysaccharide used and the extent to which it is contaminated with insoluble cellular debris. Normally, amounts of enzyme in the order of from 10—500 ppm, preferably in the range of from 50—250 ppm are used. The concentration may also be expressed in terms of the actual protease activity, as measured by Anson units, when a convenient concentration is 0.5 or 1 Au per Kg of broth. Although the enzymatic treatment can be carried out without stirring, it is preferred to stir the fermentation broth in order to minimise time and to avoid settling of solids. The enzymatic treatment can be carried out conveniently at ambient temperature or slightly above. The enzymatic treatment is often more efficient when it is carried out at moderately elevated temperatures, i.e. temperatures up to 70°C. Especially when "Esperase" is used as the enzyme, the enzymatic treatment can be carried out advantageously at a temperature between 50°C and 70°C, preferably between 60°C and 65°C. Temperatures above 70°C should be avoided since rapid degradation of the cell-lytic enzymes is likely to occur. For optimum cell-degradation activity, the aqueous polysaccharide solution is suitably maintained at a pH of between about 2 and about 11 and preferably at a pH between 4 and 10. Good results have been obtained using "Esperase" and "Maxatase" as the enzyme at a pH of about 9, and about 7, respectively.

The process according to the present invention may be carried out batchwise or continuously. When the ultrafiltration is combined with an enzymatic treatment, it is preferred to carry out the overall process continuously, when it can conveniently be effected by adding the enzyme-containing solution to a reservoir or tank containing the aqueous polysaccharide solution as diluted or undiluted fermentation broth, or, if desired, as an aqueous solution made up from unclarified commercially available or isolated polysaccharide, e.g Xanthan gum. The reaction vessel should be sized sufficiently and the rate of enzyme and polysaccharide addition should be such that the aqueous solution containing solid cellular debris is given sufficient residence time in the presence of the appropriate enzyme to afford the desired degree of cell degradation. If desired, the vessel may be heated to the temperature at which the enzyme achieves optimal activity. Upon completion of the reaction period, the enzymatically treated polysaccharide solution is transferred to the ultrafiltration unit in order to obtain concentrated product which can be either used as such or which may undergo, if desired, a further clarification treatment, e.g. by contacting at least part of the product with solid siliceous material as described hereinafter. It is also possible to recycle at least part of the concentrated solution obtained at any stage during the ultrafiltration to the vessel wherein the enzymatic treatment is carried out. This has the advantage that cellular debris not yet degraded is treated once again with the enzyme, already present or added during the residence time of the cellular debris.

As will be clear from the foregoing description, the advantages of the concentrated aqueous solution of

---

* names between inverted commas in this patent application are or are believed to be registered trade marks.

3

polysaccharide prepared by the present process lie in the transport logistics and the greater ease of use at the well-head. However, as will be readily apparent to those skilled in the art, the concentrate cannot be used as such for polymer assisted water flooding, but must be diluted to an appropriate concentration, depending on the viscosity of the flooding system required. Normally, suitable concentrations will be between 200 and 1500 ppm, especially between 500 and 1250 ppm. Although the aqueous concentrate prepared according to this invention can be diluted to such working concentrations much more readily than the polysaccharide powders hitherto available, it is nevertheless often found that efficient and homogeneous dilution of the concentrate is facilitated by incremental dilution. In one convenient embodiment of this procedure, a stream of the aqueous concentrate is metered into a stream of water which recirculates to and from a holding tank. The passage of the concentrate and water through the recirculation pump usually serves to effect satisfactory mixing, but if necessary the combined streams can be passed additionally through a static mixer before entering the holding tank.

The diluted concentrate of polysaccharide may be used directly for injection into permeable subsurface formations, e.g. in waterflooding operations, but it is often found desirable to provide a final clarification treatment to the diluted solution, suitably by filtration with a filter aid such as diatomaceous earth. If desired, such a clarification stage can be included in the preparation of the concentrate; i.e. the initial dilute broth may be subjected to a composite process incorporating enzymatic cell disruption, filtration over diatomaceous earth and ultrafiltration concentration. Alternatively, any residual cell debris could also be removed by microfiltration, i.e. by passage through a porous membrane with pore size 0.2 to 0.8 microns. In order to minimize membrane clogging, the fluid flow should be tangential to the membrane, as in "Pellicon" millipore filter cassette. The system is somewhat analogous to that used in ultrafiltration, with the major difference—in the present context—that the desired product from the microfiltration step is the cell-free permeate (i.e. filtrate), whereas in the ultrafiltration step the desired product is the concentrated retentate—the permeate being essentially water. However, although such a composite process can yield a product requiring minimal treatment at the well-head (e.g. only dilution), in general the increasing viscosity of the concentrating ultrafiltration retentate makes it difficult to maintain as effective flux through a flow bed or membrane.

The invention is illustrated in the following examples:

Example 1

To illustrate the effect of an enzymatic treatment on polysaccharide solutions, optical densities were measured (together with Brookfield viscosities determined at 25°C at a clear rate of 6.28 $sec^{-1}$) for a fermentation broth (ex TNO; "TNO" stands for "Netherlands Organization for Applied Scientific Research") as such and after treatment with "Esperase" (200 mg/kg broth) at 52°C and pH 9.4. The optical density of the untreated solution amounted to 2.7 (abs/cm) whereas the optical density of the "Esperase"-treated solution amounted to 0.13 after 1 hour, which value remained constant for at least the next hour. Treatment of another sample of the same fermentation broth with "Maxatase" (402 mg/kg broth) at 52°C and pH 7.25 showed an optical density of 2.1 after 1 hour and of 1.8 after 2 hours. The viscosity of the untreated fermentation broth (1900—2200 mPa.s) did not change substantially during the observations.

Example 2

To illustrate the reduced amount of nitrogen (originating from cellular debris) in the biopolymer after a combined enzymatic/concentration treatment a nitrogen analysis was carried out using samples both of the biopolymer and of various permeates obtained during ultrafiltration. The experiments were carried out using a fermentation broth (ex TNO) having a Brookfield viscosity of about 370 mPa.s (measured at 25°C at a shear rate of 6.28 $sec^{-1}$) and an optical density of 2.6 (abs/cm). From nitrogen analysis it appeared that the nitrogen content of the concentrate of biopolymer obtained from non-enzyme treated fermentation broth amount to 6.8% wt whereas that of an "Esperase"-treated fermentation broth amounted to 5% wt, indicating that the original nitrogen amount had decreased considerably. The nitrogen content of the various permeates decreases radidly to values below 0.01% wt. The viscosity of the "Esperase"-treated fermentation broth was still in the order of 3300 mPa.s and the optical density had become 0.17 (abs/cm). Similar effects were observed when using a "Maxazyme"-treated fermentation broth was even higher than that of the starting fermentation broth. Nitrogen amount and optical density showed similar patterns as observed for the Esperase-treated fermentation broth.

Example 3

The filterability of several fermentation broths was measured, both with and without ultrafiltration and/or enzymatic treatment using a pressure cylinder under a positive pressure of 1 bar. "Millipore" filters of 1.2 μm and 0.45 μm were used, respectively. The polysaccharide concentrates obtained after ultrafiltration were diluted with permeate to 1.4% wt and subsequently diluted with distilled water to a concentrated of 1000 ppm. For comparative purposes filterability tests were carried out using a "Xanflood" solution (A), a fermentation broth (ex TNO) (B), both without any concentration treatment and an "Esperase"-treated fermentation broth (ex TNO) without ultrafiltration (UF)-treatment (C). The filterability data, expressed as total filtrate obtained after certain periods of time at the applied pore size, are given in Table 1 below.

4

TABLE 1

| Fermentation broth (1000 ppm) | Treatment | Total filtrate (ml) after time (min) | | | |
|---|---|---|---|---|---|
| | | 1.2 µm pore | | 0.45 µm pore | |
| | | min | ml | min | ml |
| A | — | .5 | 62 | .5 | 2.9 |
| | | 1 | 95 | 4 | 13 |
| B | — | .25 | 42 | 1 | 13 |
| | | 1 | 102 | 10 | 47 |
| C | "Esperase" | .5 | 180 | 1 | 40 |
| | | | | 4 | 90 |
| B | UF | .25 | 172 | 1 | 68 |
| | | .5 | 308 | 10 | 82 |
| B | UF+"Esperase" | .25 | 155 | 1 | 105 |
| | | .5 | 282 | 4 | 286 |

Example 4

A De Danske Sukkerfabrikker (DDS) Lab. Module ultrafiltration unit and pump was set up according to the manufacturer's instructions using a GR 50P type membrane having a nominal cut-off at mol. wt. 65,000. A 1% aqueous solution of the commercial xanthan product "Kelzan" MF was recirculated through this unit at a rate sufficient to maintain an inlet pressure between 7 and 15 bar (gauge). The xanthan concentration was determined both in the permeate and in the recirculating retentate; in the permeate it was negligible, whilst in the retentate it increased from the initial 1% to a final value of 18%.

Example 5

Xanthan-containing aqueous broths resulting from the fermentation of *Xanthomonas* sp. were obtained from a variety of sources and subjected to ultrafiltration concentration using a DDS module (type 35—2,25) installed in a 200 l test circuit. The module had 7 membrane plates connected in parallel, giving a total membrane area of 1.05 m$^2$, and the membrane was that supplied by DDS under reference GR5, being a polysulphone membrane with a molecular weight cut off at 60,000. In certain instances a cell-lytic enzyme was added to the fermentation broth to break up the cellular debris. The pressure differential over the membrane was increased, and the pumping rate decreased, as concentration progressed and the results obtained are shown in Table 2 below from which it can be seen that the polysaccharide concentration can be increased to over 10% by weight.

| Exp | Feed | Pressure | | Pumping rate m/s | Biopolymer concentration in retentate %w | Flux Kg/m². day |
|---|---|---|---|---|---|---|
| | | In Bar(g) | Out Bar(g) | | | |
| 1 | Untreated Xanthomonas broth, containing 5000 ppm formaldehyde | 2.1 | 1.0 | 0.60 | 2.6 | 537 |
| | | 2.4 | 1.0 | 0.53 | 3.9 | 569 |
| | | 3.3 | 1.0 | 0.48 | 5.8 | 377 |
| | | 3.3 | 0.6 | 0.46 | 7.1 | 329 |
| | | 3.6 | 0.6 | 0.45 | 9.5 | 206 |
| 2 | Concentrate from Exp 1 diluted with tap water to initial Xanthan concentration | 2.0 | 1.0 | 0.52 | 2.0 | 507 |
| | | 2.2 | 1.0 | 0.52 | 4.1 | 507 |
| | | 2.6 | 1.0 | 0.51 | 5.2 | 366 |
| | | 3.6 | 1.0 | 0.43 | 7.6 | 293 |
| 3 | Concentrate from Exp 2 diluted with tap water to initial Xanthan concentration, and Esperase treated at the rate of 1 Anson unit per Kg broth | 1.7 | 1.0 | 0.51 | 2.8 | 510 |
| | | 2.1 | 1.0 | 0.38 | 5.7 | 391 |
| | | 2.7 | 1.0 | 0.36 | 7.6 | 302 |
| | | 3.7 | 1.0 | 0.28 | 10.4 | 137 |
| 4 | Xanthomonas broth treated with "Maxatase" (0.50 Au/Kg) | 3.8 | 3.0 | 0.4 | 1.80 | 225 |
| | | 3.8 | 2.5 | 0.4 | 3.32 | 263 |
| | | 4.6 | 2.5 | 0.4 | 5.24 | 295 |
| | | 5.5 | 3.0 | 0.4 | 6.30 | 259 |

# 0 049 012

## Claims

1. Process for the concentration of an aqueous solution of water-soluble pseudoplastic polysaccharide having a viscosity of at least 1900 mPa.s (determined at 25°C), which comprises ultrafiltration of such a solution by contacting the solution with a porous membrane having a molecular weight cut-off of at most 65,000, at a pumping rate of at least 0.28 m/s and at a pressure differential over said membrane of 2—20 bar (g), and withdrawing liquid which has permeated through the membrane.

2. Process as claimed in claim 1, in which the pumping rate is not more than 0.60 m/s.

3. Process as claimed in claim 1 or 2, wherein the membrane has a molecular weight cut-off of not more than 20,000.

4. Process as claimed in any one of the claims 1—2 wherein the polysaccharide solution has a viscosity of at least 7000 mPa.s (determined at 25°C).

5. Process as claimed in any one of the claims 1 to 4 wherein the polysaccharide solution is recirculated over the porous membrane in a direction parallel to the plane of the membrane.

6. Process as claimed in any one of the claims 1—5 wherein the aqueous polysaccharide solution is the broth resulting from fermentation of a pseudoplastic polysaccharide-producing microorganism.

7. Process as claimed in claim 6 wherein a cell-lytic enzyme is added to the fermentation broth either before or after the ultrafiltration.

8. Concentrated aqueous retentate of a water-soluble pseudoplastic polysaccharide.

## Revendications

1. Procédé pour la concentration d'une solution aqueuse de polysaccharide pseudoplastique soluble dans l'eau ayant une viscosité d'au moins 1900 mPa.s (déterminée à 25°C), qui comprend l'ultrafiltration de cette solution par mise en contact de la solution avec une membrane poreuse ayant un poids moléculaire de coupure d'au moins 65 000, à une vitesse de pompage d'au moins 0,28 m/s et avec une différence de pression sur la membrane de 2—20 bars (pression manométrique), et l'évacuation du liquide qui a passé à travers la mambrane.

2. Procédé selon la revendication 1, dans lequel la vitesse de pompage n'est pas supérieure à 0,60 m/s.

3. Procédé selon la revendication 1 ou 2, dans lequel la membrane a un poids moléculaire de coupure de pas plus de 20 000.

4. Procédé selon l'une quelconque des revendications 1—2, dans lequel la solution de polysaccharide a une viscosité d'au moins 7000 mPa.s (déterminée à 25°C).

5. Procédé selon l'une quelconque des revendications 1 à 4, dans lequel on fait circuler la solution de polysaccharide sur la membrane poreuse dans une direction parallèle au plan de la membrane.

6. Procédé selon l'une quelconque des revendications 1 à 5, dans lequel la solution aqueuse de polysaccharide est le bouillon résultant de la fermentation d'un microorganisme producteur de polysaccharide pseudoplastique.

7. Procédé selon la revendication 6, dans lequel une enzyme cellulytique est ajoutée au bouillon de fermentation avant ou après l'ultrafiltration.

8. Produit de rétention aqueux concentré d'un polysaccharide pseudoplastique soluble dans l'eau.

## Patentansprüche

1. Verfahren zum Konzentration einer wäßrigen Lösung eines wasserlöslichen pseudoplastischen Polysaccharid mit einer Viskosität von zumindest 1900 mPa.s (bestimmt bei 25°C), umfassend die Ultrafiltration einer solchen Lösung durch Zusammenbringen der Lösung mit einer porösen Membran mit einer Molekulargewichtsabtrennung von höchstens 65000 bei einer Pumpgeschwidigkeit von zumindest 0,28 m/s und einer Druckdifferenz über die Membran von 2 bis 20 bar (g) und Abziehen von Flüssigkeit, die durch die Membran hindurch gegangen ist.

2. Verfahren nach Anspruch 1, wobei die Pumpgeschwindigkeit nicht mehr als 0,60 m/s beträgt.

3. Verfahren nach Anspruch 1 oder 2, wobei die Membran eine Molekulargewichtsabtrennung von nicht mehr als 20000 ergibt.

4. Verfahren nach einem der Ansprüche 1 oder 2, wobei die Polysaccharidlösung eine Viskosität von zumindest 7000 mPa.s (bestimmt bei 25°C) aufweist.

5. Verfahren nach einem der Ansprüche 1 bis 4, wobei die Polysaccharidlösung über die poröse Membran zurückgeführt wird in einer Richtung, die parallel ist zu der Ebene der Membran.

6. Verfahren nach einem der Ansprüche 1 bis 5, wobei die wäßrige Polysaccharidlösung, die bei der Züchtung eines pseudoplastisches Polysaccharid-bildenden Mikroorganismus anfallende Fermentationsbrühe ist.

7. Verfahren nach Anspruch 6, wobei ein Zell-lösendes Enzym zu der Fermentationsbrühe zugesetzt wird, entweder vor oder nach der Ultrafiltration.

8. Konzentrierte wäßrige (bei der Ultrafilatration) zurückgehaltene Lösung eines wasserlöslichen pseudoplastischen Polysaccharids.